**Europäisches Patentamt**

(19) **European Patent Office**

**Office européen des brevets**

(11) Veröffentlichungsnummer: **0 016 357**
**B1**

(12) # EUROPÄISCHE PATENTSCHRIFT

(45) Veröffentlichungstag der Patentschrift:
26.05.82

(51) Int. Cl.³: **C 07 C 143/34**

(21) Anmeldenummer: **80100944.0**

(22) Anmeldetag: **26.02.80**

(54) Verfahren zur Herstellung von Erdalkalisalzen von Alkylbenzolsulfonsäuren.

(30) Priorität: **02.03.79 DE 2908265**

(43) Veröffentlichungstag der Anmeldung:
**01.10.80 Patentblatt 80/20**

(45) Bekanntmachung des Hinweises auf die Patenterteilung:
**26.05.82 Patentblatt 82/21**

(84) Benannte Vertragsstaaten:
**AT BE DE FR GB IT NL SE**

(56) Entgegenhaltungen:
**GB-A-1 172 779**
**US-A-3 719 596**

(73) Patentinhaber: **HOECHST AKTIENGESELLSCHAFT,
Postfach 80 03 20, D-6230 Frankfurt/Main 80 (DE)**

(72) Erfinder: **Wellbrock, Werner, Dr., Drei Linden Strasse 30,
D-6232 Bad Soden am Taunus (DE)**

## Verfahren zur Herstellung von Erdalkalisalzen von Alkylbenzolsulfonsäuren

Erdalkalisalze von Alkylbenzolsulfonsäuren finden technisch im grossen Umfange Verwendung zur Bereitung von Emulsionskonzentraten wasserunlöslicher Biocide, besonders für Insekticide und Herbicide. Die für diesen Zweck verwendeten Produkte müssen im wesentlichen wasserfrei sein. Die Herstellung dieser Verbindungen erfolgt in bekannter Weise nach einem 2stufigen Verfahren. Dabei wird zunächst aus der Alkylbenzolsulfonsäure, die nach üblichen Sulfonierungsverfahren aus Alkylbenzolen mit etwa 9 bis 18 Kohlenstoffatomen in den Alkylresten erhalten wird, im wasserhaltigen Medium das gewünschte Erdalkalisalz, vornehmlich das Calciumsalz hergestellt. Da es bisher nicht gelungen ist, die Neutralisation von Alkylbenzolsulfonsäuren mit Erdalkalihydroxid, insbesondere mit Calcium- oder Bariumhydroxid, in Abwesenheit von Wasser vorzunehmen, wird sie in einem wasserhaltigen organischen Lösungsmittel, im allgemeinen wasserhaltige niedere Alkohole, insbesondere Isobutanol, durchgeführt. Aus der erhaltenen neutralen Lösung der Alkylbenzolsulfonate in dem wasserhaltigen Lösungsmittel wird sodann durch azeotrope Destillation das Wasser und grosse Teile des organischen Lösungsmittels entfernt.

Es wurde nun gefunden, dass man auf den Zusatz des Wassers verzichten kann, wenn man die Neutralisation in Anwesenheit geringer Mengen eines Oxalkylats durchführt.

Gegenstand der Erfindung ist somit ein Verfahren zur Herstellung von Erdalkalisalzen, insbesondere Calciumsalzen, von Alkylbenzolsulfonsäuren durch Neutralisation der entsprechenden freien Alkylbenzolsulfonsäuren, das darin besteht, dass man eine Lösung der Alkylbenzolsulfonsäure in einem organischen Lösemittel mit 1 bis 10, vorzugsweise 1 - 5 Gew.-% eines Oxalkylats, bezogen auf das Gewicht der Alkylbenzolsulfosäure, versetzt, durch Zugabe der erforderlichen Menge einer basischen Erdalkalimetall-Verbindung neutralisiert und das entstandene Salz der Alkylbenzolsulfosäure isoliert.

Das erfindungsgemässe Verfahren wird im allgemeinen so durchgeführt, dass man das Lösemittel vorlegt, das Neutralisationsmittel und das Oxalkylat zugibt, auf eine Temperatur von ca. 70°C - 90°C erhitzt und dann die Alkylbenzolsulfosäure zugibt. Nach Beendigung der Neutralisation, was durch Überprüfung des pH-Werts festgestellt werden kann, wird das entstandene Alkylbenzolsulfonat durch Filtration isoliert. Die Zugabe der einzelnen Produkte zu dem organischen Lösungsmittel kann auch in veränderter Reihenfolge geschehen, doch ist die oben geschilderte Verfahrensweise bevorzugt.

Um das bei der Neutralisation entstehende Reaktionswasser zu entfernen, wird nach beendeter Neutralisation dieses Wasser zusammen mit einem Teil des Lösemittels azeotrop abdestilliert. Diese Destillation kann vor oder nach der Filtration erfolgen, je nachdem, ob das Phenylsulfonat in fester Form oder als wasserfreie Einstellung in dem organischen Lösemittel anfallen soll.

Als Lösemittel für dieses Verfahren kommen niedere Alkohole, insbesondere i-Butanol und i-Propanol in Frage, aber auch aromatische Lösungsmittel wie p-Xylol, Benzol, Toluol und auch Mineralöle. Bei den Alkylbenzolsulfosäuren handelt es sich um die Monosulfosäuren von Alkylbenzolen, die 8 bis 18 C-Atome in den Alkylresten aufweisen.

Als Oxalkylate können Produkte aus folgenden Verbindungsklassen verwendet werden: oxäthylierte Triglyceride mit 20 - 60 Einheiten Äthylenoxid, vorzugsweise Ricinusöl mit 36 - 40 Einheiten Äthylenoxid; oxalkylierte $C_{12}$-$C_{20}$-Fettalkohole bzw. Fettsäuren mit jeweils 4 - 30 Einheiten Äthylenoxid; oxalkylierte Alkylphenole mit 8 bis 18 C-Atomen in den Alkylketten und 8 bis 30 Einheiten Äthylenoxid sowie Polyäthylenglykolmethyl- oder -äthyläther mit 1 bis 8 Einheiten Äthylenglykol. Als Neutralisationsmittel dienen basische Verbindungen der Erdalkalimetalle, vorzugsweise Calciumhydroxid, -carbonat oder -oxid.

Das oben beschriebene Verfahren bietet den Vorteil, dass man bei Neutralisation auf die Zugabe von Wasser verzichten kann und auf diese Weise Zeit und Energie spart.

Beispiel 1

813 ml Xylol werden vorgelegt und unter Rühren 30 g Ca(OH)₂ zugegeben. Man rührt 10 Minuten, gibt dann 16 g Diethylenglykoldimethylether zu und heizt auf 70°C. Innerhalb von 3 Stunden werden dann 250 g Dodecylbenzolsulfosäure zugegeben bis ein pH Wert von 4 erreicht wird. Der Ansatz wird noch 30 Minuten nachgerührt und dann weiter aufgeheizt bis zu einer Innentemperatur von 142°C. Dabei destillieren ca. 200 ml eines Xylol-Wasser-Gemischs ab. Der Kesselinhalt wird auf 70°C abgekühlt, 30 Minuten nachgerührt und abfiltriert. Man erhält ca. 800 g des Calciumsalzes der Dodecylbenzolsulfosäure.

Beispiel 2

Zu 100 g wasserfreiem i-Butanol werden 3 g Nonylphenol + 9 AeO zugegeben und diese Mischung mit 13,5 g Ca(OH)₂ versetzt. Man heizt auf 70°C auf und gibt unter Rühren langsam 93 g Dodecylbenzolsulfosäure zu bis ein pH-Wert von ca. 6 erreicht ist. Es wird eine Stunde nachgerührt und das Calciumsalz abfiltriert.

Das bei der Neutralisation entstandene Wasser wird azeotrop abdestilliert. Insgesamt werden dabei ca. 72 ml i-Butanol entfernt, die das gesamte Wasser enthalten. Man erhält 140 g wasserfreies Calcium-Dodecylbenzolsulfonat (70%-ig).

## Patentansprüche

1. Verfahren zur Herstellung von Erdalkalisalzen von Alkylbenzolsulfonsäuren durch Neutralisation von Alkylbenzolsulfosäuren, dadurch gekennzeichnet, dass man die Alkylbenzolsulfonsäure in einem organischen Lösungsmittel und in Gegenwart von 1 bis 10 Gew.-% eines Oxalkylats, bezogen auf das Gewicht der Alkylbenzolsulfosäure mit der erforderlichen Menge einer basischen Erdalkalimetallverbindung neutralisiert.

2. Verfahren nach Anspruch 1, dadurch gekennzeichnet, dass man zu dem organischen Lösungsmittel zunächst das Oxalkylat und die basische Erdalkalimetall-Verbindung und dann die Alkylbenzolsulfonsäure zugibt.

## Claims

1. Process for the preparation of alkaline earth metal salts of alkyl benzene sulfonic acids by the neutralization of alkyl benzene sulfonic acids, which comprises neutralizing the alkyl benzene sulfonic acid with the necessary amount of a basic alkaline earth metal compound in an organic solvent and in the presence of 1 to 10% by weight of an oxalkylate, calculated on the weight of the alkyl benze sulfonic acid.

2. A process as claimed in claim 1 which comprises adding first the oxalkylate and the basic alkaline earth metal compound to the organic solvent and then adding the alkyl benzene sulfonic acid.

## Revendications

1. Procédé de préparation de sels de métaux alcalinoterreux d'acides alkyl-benzène-sulfoniques par neutralisation d'acides alkyl-benzène-sulfoniques, procédé caractérisé en ce qu'on neutralise l'acide alkyl-benzène-sulfonique, dans un solvant organique et en présence de 1 à 10% en poids d'un produit d'alcoxylation, par rapport au poids de l'acide alkyl-benzène-sulfonique, avec la quantité nécessaire d'un composé basique de métal alcalinoterreux.

2. Procédé selon la revendication 1, caractérisé en ce qu'on ajoute, au solvant organique, d'abord le produit d'alcoxylation et le composé basique de métal alcalinoterreux, puis l'acide alkyl-benzène-sulfonique.